# EUROPEAN PATENT APPLICATION

(11) **EP 0 650 735 A2**
(43) Date of publication of application: **03.05.1995**
(21) Application number: 94201932.4
(22) Date of filing: 05.07.1994
(51) Int. Cl.: A61K 47/48, A61K 39/395, A61K 51/10

(54) **Kit and method for pretargeting**

(30) Priority: 09.07.1993 EP 93202016
(71) Applicant: Akzo Nobel N.V., NL-6824 BM Arnhem (NL)
(72) Inventor: Bos, Ebo Sybren, NL-5344 JA Oss (NL); McCabe, Richard P., West Chester, Pennsylvania 19830 (US)
(74) Representative: Mestrom, Joannes Jozef Louis

(57) **Abstract**

The invention describes a novel kit or method for the delivery of pharmaceutical or diagnostic compounds to target cells in the body. This kit comprises a monospecific antibody with multiple binding sites, which antibody recognizes a target associated antigen, and a ligand-effector moiety, of which the ligand has been derived from the target associated antigen or analogue thereof. Specifically the invention relates to targeting cytotoxic drugs or radioisotopes to tumor cells. The preferred antibody is a human IgM and the ligand is preferably the epitope cognate to said antibody.

SEQUENCE LISTING

(1) GENERAL INFORMATION:

(i) APPLICANT:
(A) NAME: Akzo Nobel N.V.
(B) STREET: Velperweg 76
(C) CITY: Arnhem
(E) COUNTRY: The Netherlands
(F) POSTAL CODE (ZIP): 6824 BM
(G) TELEPHONE: 04120-66911
(H) TELEFAX: 04120-50592
(I) TELEX: 37503 akpha nl

(ii) TITLE OF INVENTION: Kit and method for pretargeting

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: EP 93.202.016.7
(B) FILING DATE: 09-JUL-1993

(2) INFORMATION FOR SEQ ID NO: 1:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(v) FRAGMENT TYPE: internal

(xi) SEQUENCE DESCRIPTION: SEQ ID NO: 1:

Thr Leu Gln Gly Leu Glu Ile Glu Leu Gln Ser Gln Leu Ser Met Lys
1               5                   10                  15

## Description

The invention relates to the fields of immunotherapy and immunodiagnostics, more particular to the field of pretargeting. The invention describes a kit and method for the delivery of pharmaceutical compounds or diagnostics to target cells in the body. These kits comprise an antibody or a fragment thereof, or a derivative of said antibody or fragment, which antibody or fragment recognize a target associated antigen, and a ligand-effector moiety conjugate comprising a ligand specific for said antibody, and an effector moiety linked to the ligand.

The in vivo use of antibodies for the selective delivery of diagnostic or therapeutic agents to target tissues is known in the art.

Since the advent of methodology for the production of monoclonal antibodies, antibodies of predefined specificities (Köhler and Milstein, Nature 256, 495-497, 1975), numerous studies aiming at specific targeting of radionuclides, plant or bacterial toxins, biological agents and cytostatic agents (drugs) to target cells or tissues with the help of said antibodies have been performed, as reviewed by Bator and Reading (in: Therapeutic Monoclonal Antibodies, eds: C.A.K. Borrebaeck and J.W. Larrick, Stockton Press, pp. 35-56, 1990). In in vitro studies the great potential of these immunoconjugates has been amply demonstrated. Application of such immunoconjugates in vivo in tumor bearing animals as well as in cancer patients has met with much less success. A major problem has been the inability to attain a sufficient difference in uptake of the immunoconjugates between the target tissues as compared to the non-target tissues. As a result it has been difficult for instance to effect an increase in therapeutic index of clinically used anti-cancer drugs by administration in the form of a drug-immunoconjugate. Similarly, due to the lack of operational selectivity of therapeutic radio-immunoconjugates when administered in vivo, normal tissues are unduly exposed to destructive radiation. The therapeutic and diagnostic limitations of immunoconjugates, apart from a number of factors adversely affecting antibody delivery, are partly due to changes in antibody affinity, specificity and immunogenicity upon chemically linking a diagnostic or therapeutic agent to an antibody.

In order to overcome part of the problems mentioned, newer approaches to delivery of diagnostic or therapeutic agents have recently been suggested, that are characterised in that the localisation phase of the antibody component is separated in time from the phase in which the diagnostic or therapeutic agent is administered. In these so-called pretargeting schemes sufficient time is permitted for accumulation of non-labelled antibody at the target site and for elimination of the antibody from other non-target tissues. The latter process, the clearing phase, may be aided by, for example, plasmaphoresis or use of a second antibody reactive with the first. At the time of optimal antibody accumulation at the target site as compared to the non-target tissue, the diagnostic or therapeutic agent to be recognised by the antibody already localised, is administered.

The pretargeting approaches which have been so far described in literature require that the antibody must have a dual specificity: a specific site that allows binding to a target tissue associated antigen and a specificity that enables specifically binding of the diagnostic or therapeutic agent.

Early on, bifunctional antibodies were constructed by chemically linking univalent fragments of antibodies with different specificities. Raso and Griffin (Cancer Res. 41, 207, 1981) for instance constructed a bifunctional antibody that was able to bind human IgG and to ricin, a toxic lectin. Later on, technology has been developed to produce hybrid hybridomas that produce monoclonal antibodies of dual specificity (Reading, US Patent 4,474,893). A biologically produced bifunctional antibody recognising both carcinoembryonic antigen (CEA) and vinca alkaloids was described by Corvalen et al. (Cancer Immunol. Immunother. 24, 133, 1987). Studies on xenografts in nude mice revealed the combined use of the bifunctional antibody and vinblastin, given 7-11 days following administration of the antibody, to be more effective than drug alone in suppressing tumor growth.

Recently pretargeting techniques have been proposed in which the specific antibody recognises a haptenic group, i.e. an EDTA derivative chelating indium (Stickney et al., Cancer Res. 51, 6650, 1991).

In a closely related domain of the prior art monovalent, target site selective antibodies have been endowed with a second specificity by linkage, through either chemical or biological methods, to a member of a specifically interacting binding pair. The high affinity system avidin-biotin has been adopted in such an approach. (European Patent No.:0 251 494, to the Board of Trustees of the Leland Stanford University). The pretargeting system used in this patent application employs an antibody that has been linked to the protein avidin or streptavidin and a biotinylated metal chelate.

A major problem with the bifunctional antibodies mentioned above, and with most other pretargeting systems described in the literature, is that the antibodies are of murine origin. These, like all other foreign proteins, are highly immunogenic in man. The phenomenon of HAMA, human anti mouse antibodies, is well known in the field and severely limits the use of mouse derived antibodies in diagnostic and especially in therapeutic applications in human beings. A single application of a murine antibody is usually sufficient to mount an immune response that will prevent subsequent applications to be effective. This immunogenicity problem will even be aggrevated when antibody-conjugates are made with non-human proteins, such as avidin.

Another problem which is inherent to the pretargeting schemes described above is that the immunoconjugates in most cases have to be made chemically, which introduces not only possible loss of specificity, but also loss in yield of the conjugate due to incomplete reactions and losses during purification of the conjugate.

The present invention provides a kit, comprising an antibody or a fragment thereof or a derivative of said antibody or fragment, which fragment or antibody recognizes a target associated antigen, and a ligand-effector moiety conjugate comprising a ligand specific for said antibody, and an effector moiety linked to the ligand, characterised in that:
- said antibody is a mono specific antibody with multiple binding sites,
- said ligand is derived from the target associated antigen or a cross-reactive analogue thereof recognisable by said antibody.

Using a monospecific antibody gives the advantage that there is no need for chemical conjugation methods.

The present invention proposes to administer to a patient a mono specific antibody in a dose, or in successive doses, sufficient to maximise target site accumulation of the antibody. At some optimal time later, when most of the non-bound antibody has been cleared, a diagnostic or therapeutic effector molecule carried by the target associated antigen or a cross-reactive analogue thereof, is administered. As target associated antigen every antigen that is specific for a population of target cells can be used. Examples of antigens which are specifically useful are: antigens related to cancer cells, like CEA (Carcino-embryonic antigen), AFP (alpha-foetoprotein), FHAP (fast homoarginine-sensitive alkaline phosphatase), p97 (melanome specific), and EL-1 (elongation factor 1), antigens related to viruses or bacteria, like HIV gp 120, HIV gp 160, Hepatitis B core antigen and Outer Membrane Protein of Borrelia burgdorferi, autoimmune related antigens, like small nuclear RNA's and Mycobacterium 65 kD HS-protein.

Preferably the ligand derived from the target associated antigen is the cognate epitope, which is recognised by the antibody. This epitope is a small, low-molecular weight part of the antigen to which the antibody or fragment of antibody specifically binds. If the antigen is a glycoprotein the epitope can be formed by a small sequence of amino acids, by the carbohydrate moieties of the protein or by a combination of those two. In the case of a sequence of amino acids this can be formed by a sequence which is linearly present in the antigen (linear epitope), but it is also possible that due to the three-dimensional conformation of the protein the epitope has been formed by a sequence of amino acids which is not linearly present in the antigen, but which, according to the folding of the protein is presented as an adjacent sequence (conformational epitope).

To be able to bind more or more efficiently to the antibody another embodiment of the invention is to present the epitope as a multimer. Multimerization can be achieved by common chemical techniques, in case of peptide epitopes it is also possible to produce a multimer by recombinant DNA techniques. In this last way the nucleotides coding for the epitope are introduced as repeating sequences in an expression system.

Next to the epitope also epitope-analogues which are cross-reactive with the epitope can be used. Cross-reactive means here that the analogue is able to compete with the epitope for binding with the antibody.

Monospecific antibodies to an epitope can be obtained by affinity purification from polyspecific antisera by a modification of the method of Hall et al. (Nature 311, 379-387, 1984). Polyspecific antisera can be obtained by immunizing rabbits according to standard immunisation schemes. Monospecific antibody as used herein is defined as a single antibody species or multiple antibody species with homogeneous binding characteristics for the relevant antigen. Homogeneous binding as used herein refers to the ability of the antibody species to bind to a specific antigen or epitope.

The antibody is preferably a monoclonal antibody, more preferably a humanised monoclonal antibody.

Monoclonal antibodies can be prepared by immunizing inbred mice, preferably Balb/c with the appropriate protein by techniques known in the art (Kohler and Milstein, Nature 256, 495-497, 1975). Hybridoma cells are subsequently selected by growth in hypoxanthine, thymidine and aminopterin in an appropriate cell culture medium such as Dulbecco's modified Eagle's medium (DMEM). Antibody producing hybridomas are cloned, preferably using the soft agar technique of MacPherson, (Soft Agar Techniques, Tissue Culture Methods and Applications, Kruse and Paterson, eds., Academic Press, 276, 1973). Discrete colonies are transferred into individual wells of culture plates for cultivation in an appropriate culture medium. Antibody producing cells are identified by screening with the appropriate immunogen. Immunogen positive hybridoma cells are maintained by techniques known in the art. Specific anti-monoclonal antibodies are produced by cultivating the hybridomas in vitro or preparing ascites fluid in mice following hybridoma injection by procedures known in the art.

It is preferred to use humanized antibodies. Methods for humanizing antibodies, such as CDR-grafting, are known (Jones et al., Nature 321, 522-525, 1986). Another possibility to avoid antigenic response to antibodies reactive with polypeptides according to the invention is the use of human antibodies or fragments or derivatives thereof.

Human antibodies can be produced by in vitro stimulation of isolated B-lymphocytes, or they can be isolated from (immortalized) B-lymphocytes which have been harvested from a human being immunized with at least one polypeptide according to the invention. This last method has been described in the European Patent Application no: 0 488 470 of Akzo N.V.

Antibodies are formed from basic Y-shaped immunoglobulin molecules, of which the variable parts are able to bind with the antigen. Five distinct classes of immunoglobulin molecules are recognized in most higher mammals, three of which (IgG, IgD and IgE) are monomeric proteins, while IgA is mostly dimeric and IgM is pentameric. Each monomer has two identical regions which can bind to the antigen, rendering the antibody monospecific.

Preferably the antibody is of the IgM type. This type of antibodies is characterized by 5 times 2 identical binding places. Multiple identical binding places enable both the attachment to more than one antigen at the target site, thus creating a more stable binding, and/or the attachment of more than one ligand-effector moieties, thus creating the opportunity to introduce more diagnostic label or therapeutic drug at the target site.

These IgM type antibodies can be obtained through cells normally producing IgM-type antibodies or they can be obtained by causing a class switch of the immunoglobulin antitype from IgG or IgA producing cells, said IgG or IgA having the desired binding characteristics.

A preferred human monoclonal IgM-type antibody is MoAb 16-88 produced by a hybridoma cell line which is deposited under number HB 8495 with the American Type Culture Collection on January 30, 1984. Reactivity of this monoclonal antibody has been specified in US Patent No. 5,106,738.

The cognate epitope for monoclonal antibody 16-88 is an epitope which is related to cytokeratines 8, 18 and 19, to which 16-88 has been demonstrated to show activity. More particularly, the epitope is a polypeptide formed by the amino acid sequence -Thr-Leu-Gln-Gly-Leu-Glu-Ile-Glu-Leu-Gln-Ser-Gln-Leu-Ser-Met-Lys- or a fragment or functional equivalent thereof.

The term "polypeptide" refers to a molecular chain of amino acids, does not refer to a specific length of the product and if required can be modified in vivo or in vitro, for example by glycosylation, amidation, carboxylation or phosphorylation; thus inter alia peptides, oligopeptides and proteins are included within the definition of polypeptide.

The term "fragment" refers to any sequence of amino acids that is part of the polypeptide defined above, having common elements of origin, structure and mechanism of action that are within the scope of the present invention because they can be prepared by persons skilled in the art, once given the teachings of the present invention.

As used herein, "functional equivalent" means variations of the described sequence still maintaining functional characteristics of the above given amino acid sequence. These functional characteristics are the ability to react with the antibody 16-88.

The variations that can occur in a sequence may be demonstrated by (an) amino acid difference(s) in the overall sequence or by deletions, substitutions, insertions, inversions or additions of (an) amino acid(s) in said sequence. Amino acid substitutions that are expected not to essentially alter biological and immunological activities, have been described. Amino acid replacements between related amino acids or replacements which have occurred frequently in evolution are, inter alia Ser/Ala, Ser/Gly, Asp/Gly, Asp/Asn, Ile/Val (see Dayhof, M.D., Atlas of protein sequence and structure, Nat. Biomed. Res. Found., Washington D.C., 1978, vol. 5, suppl. 3). Based on this information Lipman and Pearson developed a method for rapid and sensitive protein comparison (Science 227, 1435-1441, 1985) and determining the functional similarity between homologous polypeptides.

A polypeptide which shows slight variations to the polypeptide with a sequence as mentioned above is the 180-188 amino acid fragment of the 65 kD HS-protein of Mycobacterium.

The effector moiety can be either a therapeutic drug or a diagnostic compound. As a diagnostic compound preferably a γ-emitter is used, such as ^{99m}Tc, ¹¹¹In or ¹²³I (Goldenberg et al., Antib. Immunoconj. and Radiopharm., 3(3), 151-167, 1990).

When using a therapeutic drug as effector moiety preferably a compound selected from the group consisting of α- and β-emitting radioisotopes, drugs, toxins, boron addends, photosensitizers and radiosensitizers is used (Bos et al., J. Contr. Release 16, 101-112, 1991). As radioisotopes preferably ⁹⁰Y, ¹³¹I, ¹⁸⁶Re, ¹⁸⁸Re, ²¹¹At, ²¹²Bi, ²¹³Bi or ²²⁵Ac are used.

Also part of the invention are ligand-effector moieties for therapeutic use in which the ligand has been derived from a target associated antigen or a cross-reactive analogue thereof and in which the effector has been conjugated to the ligand.

Preferably the ligand which has been derived from a target associated antigen is the epitope of that antigen or a cross-reactive analogue thereof.

A preferred embodiment is the epitope that is recognised by monoclonal antibody 16-88, especially the epitope which has the amino acid sequence -Thr-Leu-Gln-Gly-Leu-Glu-Ile-Glu-Leu-Gln-Ser-Gln-Leu-Ser-Met-Lys- or a fragment or functional equivalent thereof or a multimere of this epitope. Such a multimere provides a multitude of binding places, or a multitude of sites to which the effector moiety can be conjugated. This last is especially advantageous because in such a way an effective dose of effector can be multiplied.

Numerous methods for the preparation of the ligand-effector moiety conjugates of the present invention are known in the art. Use can be made of the chemical methodology that has been developed in the field of protein-protein conjugation and antibody-drug conjugation. Overviews of these methods are given by Means and Feeney (Bioconj. Chem.1, 2-12, 1990) and Reisfeld et.al. (Antibody, Immunoconjugates and Radiopharmaceuticals 2, 217-224, 1989), which are incorporated herein by reference. For instance, the use of well known homo- or heterobifunctional protein cross-linking reagents allow the coupling of an effector moiety to a ligand by means of either a readily cleavable disulfide bond, or a more stable thioether or amide bond, or the like. A preferred general conjugation methodology makes use of a heterobifunctional reagent, such as N-succinimidyl 4-(N-maleimidomethyl)cyclohexane-1-carboxylate (SMCC), or derivatives thereof, which have a maleimido group at one end and an activated ester group at the other end. Either the ligand, or the effector moiety is derivatized with SMCC through reaction with a nucleophilic amino group, or the like. The other conjugate component, which either contains a free thiol group, or which is derivatized for instance with the well known N-succinimidyl 3-(2-pyridyldithio)propionate (SPDP) or with 2-iminothiolane (Traut's reagent) to contain a free thiol group, is reacted with the maleimido-derivatized component to give a conjugate in which the components are joined by a stable thioether bond.

When the effector moiety is to be a diagnostic or therapeutic radioisotope, the conjugation with the ligands of the invention can be performed, for instance, by the methods described by Fritzberg et.al. (Pharmaceutical Research 5, 325-334, 1988), which is incorporated herein by reference. Many of the suitable radioisotopes require conjugation through the use of bifunctional chelates, while radioiodination is preferably done employing oxidants like chloroamine-T and iodogen (Fraker and Speck, Biochem. Biophys. Res. Comm. 80, 849-857, 1978).

Another embodiment of the invention is formed by a kit in which a diagnostic moiety is coupled to the antibody. Diagnostic immunoconjugates are commonly known in the art. In this setting a diagnostic immunoconjugate is advantageous because it provides a way to investigate the biodistribution of the antibody used in the pretargeting step. Preferably the diagnostic moiety is a γ-emitting radioistope, preferably from the group of ^{99m}Tc, ¹¹¹In or ¹²³I. Coupling of the radioisotope to the antibody can be performed through known chemical methods, either by direct coupling or by coupling to bridging or linker molecules.

For pharmaceutical use the antibody and the ligand-effector conjugate should be in a pharmaceutically acceptable form.

The useful dosage to be administered will vary depending on the age, weight and mode of administration.

Preferably the pharmaceutical compound is mixed with one or more pharmaceutically acceptable carriers, e.g. as described in the standard reference Chase et al., Remington's Pharmaceutical Sciences. Examples of pharmaceutically acceptable carriers or diluents useful in the present invention include stabilizers such as SPGA, carbohydrates (e.g. sorbitol, mannitol, starch, sucrose, glucose, dextran), proteins such as albumin, tetanus toxoid, keyhole limpet haemocyanin or casein, protein containing agents such as bovine serum or skimmed milk and buffers (e.g. phosphate buffer).

By means of pharmaceutically suitable liquids the compound can also be applied as an injection preparation in the form of a solution, suspension, emulsion, or as a spray, e.g. a nasal spray.

Kits and conjugates according to the present invention can be used for the treatment and detection of tissue specific cell disorders such as cancer, auto-immune diseases and inflammatory diseases.

The invention further comprises a method for pretargeting, this method being characterized in that an antibody or a fragment thereof or a derivative of said fragment or antibody is administered followed by a subsequent administration of a ligand-effector moiety, further characterized in that said fragment or antibody is monospecific and that said ligand is derived from the target-associated antigen or a cross-reactive analogue thereof recognisable by said antibody. Furthermore all specific embodiments mentioned above apply for use in such a method for pretargeting.

The present invention is further described by way of example with reference to the accompanying figures, in which:
fig. 1 shows the pretargeting of a conjugate with biotin and the 180-188 peptide of the 65 kD HS-protein of Mycobacterium, conjugated involving an intermediate with NHS. Specific reaction with increasing concentrations of antibody 16-88 is shown;
fig. 2 shows the same pretargeting as fig. 1 but now the conjugate is made involving an intermediate with LC-SPDP. Specificity for antibody 16-88 is preserved;
fig. 3 shows an autoradiogram of ¹²⁵I-βhCG bound to IgM 593B and not to control antibody IgM SPM5B;
fig. 4 shows the radioactivity count of fractions eluted from a Sepharose column. The amount of radioactivity from ¹²⁵I-βhCG is related to a peak in the concentration of IgM 593B.

### EXAMPLE 1

### 1. Pretargeting with IgM 16.88, with biotin as effector molecule.

### 1.1 Synthesis of peptide-biotin conjugate.

The peptide TFGLQLELT (amino acid 180-188 of the 65 kD HS-protein of Mycobacterium) was solved in 50 µl DMSO (dimethylsulphoxide) in a concentration of 30 mMol/l. Next, 50 µl of a 300 mMol/l solution of biotin-NHS conjugate (Pierce) was mixed with the peptide solution and incubated for 1 hour at room temperature. Then 1.0 ml 100% ethanol was added and the solution was vortexed. The mixture was centrifuged fro 2 minutes 1400 rpm and the pellet was washed twice with 1.0 ml 100% ethanol. PBS (7.65 g/l NaCl, 0.21 g/l KH₂PO₄, 0.91 g/l Na₂HPO₄.2H₂O, pH 7.2) was used to dissolve the pellet to a final concentration of ca. 1 mg/ml of the peptide-biotin conjugate.

### 1.2 Coating of microtiter plates.

The peptide Thr-Leu-Gln-Gly-Leu-Glu-Ile-Glu-Leu-Gln-Ser-Gln-Leu-Ser-Met-Lys was dissolved in PBS to a concentration of 1 µg/ml. Microtiter wells were filled with 110 µl of this solution per well and incubated for 1 hr at 37°C. After incubation the plate was washed three times with PBS/Tween and three times with PBS 250 µl/well. The wells were blocked with 250 µl/well of 5% skimmed milk (Difco) in PBS/Tween and incubated for 1 hr at 37°C. After incubation the plate was washed three times with PBS/Tween and three times with PBS 250 µl/well.

### 1.3 Pretargeting

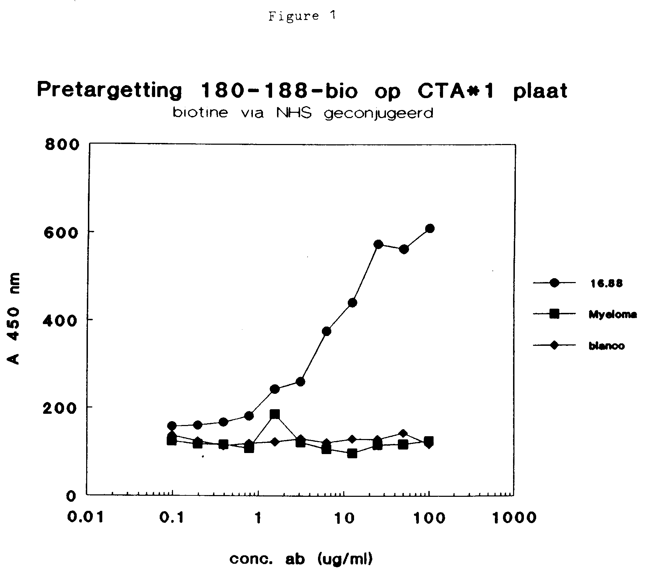

To the coated wells of a plate prepared as described above various concentrations of antibody 16-88 or control antibody were added and incubated for 1 hr at room temperature. After a wash step as described above the wells were incubated for 1 hr at room temperature with a 20 µg/ml solution of the peptide-biotin conjugate dissolved in PBS (from Ex. 1.1) in the presence of 1% BSA (bovine serum albumin). After a wash step the wells were incubated for 1 hr at room temperature with a 1:1000 solution of Streptavidin-HRP (Streptavidin-Horse Radish Peroxidase, Sanbio) in PBS and 1% BSA. After a wash step the wells were incubated with 100 µl/well TMB-substrate (10.2 ml MQ, 0.2 ml TMB, 1.08 ml buffer and 0.12 ml UPO; Organon Teknika). Next the colouring reaction was stopped with 4M H₂SO₄ 50 µl/well. Colour intensities were measured by absorption spectrometry at 450 nm. Results are depicted in fig. 1.

### Example 2

### 2. Pretargeting of Example 1, conjugates made through SPDP.

### 2.1 Synthesis biotin-HPDP and 180-188-LC-SPDP conjugates.

180-188 peptide and LC-SPDP (Pierce) were dissolved in small volumes of DMSO and mixed in a proportion of 1 part peptide to 1.5 part LC-SPDP. This mixture was incubated for 30 minutes at room temperature, then 1 M DDT was added to a final concentration of 10 mM. After again 30 minutes the peptide was precipitated with 1 ml 100% ethanol. The pellet was washed twice with ethanol and dissolved in a small volume of DMSO.
Biotin-HPDP (Pierce) was dissolved in DMSO and added to the solution of peptide-LC-SPDP in a 10:1 proportion. After 60 minutes the peptide-biotin conjugate was precipitated with 1 ml 100% ethanol, washed twice with ethanol and dissolved in PBS.

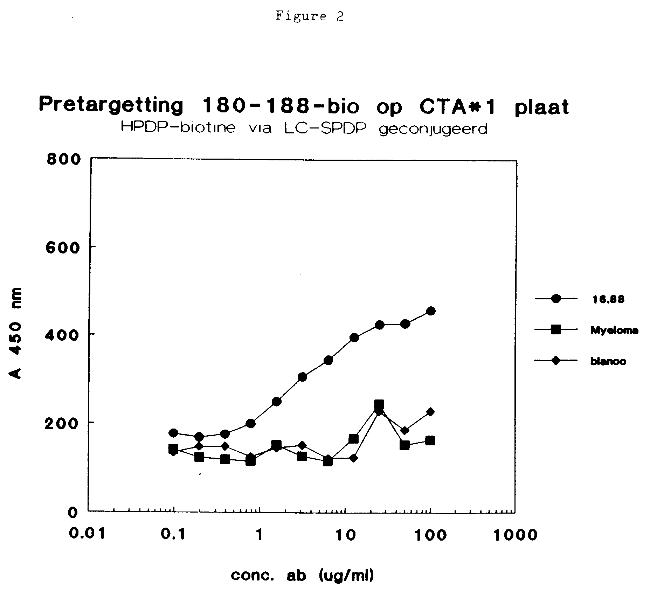

Pretargeting and enzyme-immunoassay were performed as in Example 1. Results are depicted in fig. 2.

### EXAMPLE 3

### 3. Pretargeting with βhCG and antibody to βhCG.

### 3.1 Binding of ¹²⁵I-βhCG to IgM 593B.

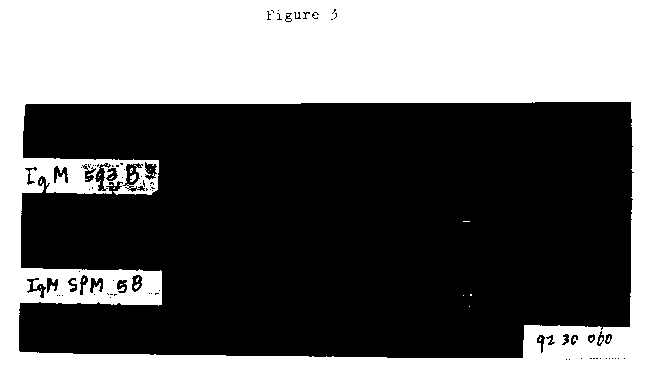

A range of concentrations of IgM 593B and a control antibody (SPM5B) were spotted on a spotblot (Immobilon PVDF membrane). After addition with Blotto the spots were incubated with 20 µg/ml ¹²⁵I-βhCG in PBS for 1 hour at room temperature. After a washstep the blot was autoradiographed. Results are depicted in fig. 3.

### 3.2 Binding of IgM 593B to hCG-Sepharose.

hCG 555 (which contains 40% hCG) was conjugated to tresyl activated Sepharose. To an amount of hCG-Sepharose equivalent with 1 mg hCG excess IgM 593B (20mg in PBS) was added. After incubation for 15 hr at 4°C and a wash step the extinction at 280 nm was measured. It appeared that 1 mg IgM 593B had been bound. In a control experiment with IgM SPM5B only 0.03 mg was bound.

### 3.3 Pretargeting.

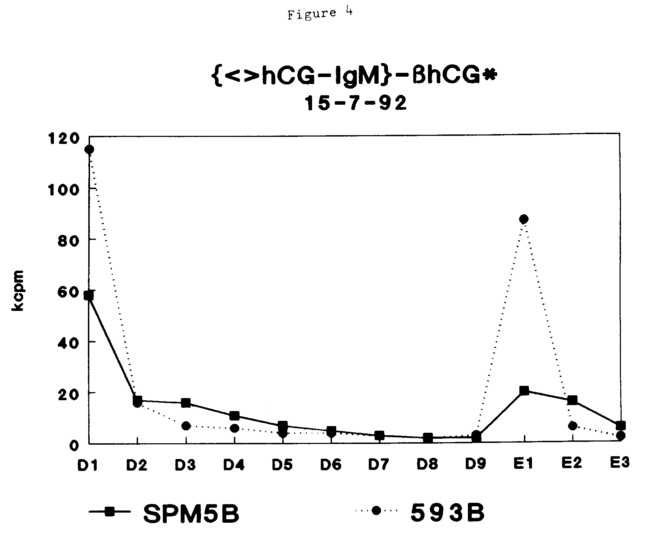

5 µg ¹²⁵I-βhCG in 0.5 ml PBS was added to hCH-Sepharose complexes loaded with IgM 593B as described above. Free ¹²⁵I-βhCG was removed by washing with PBS. hCG-Sepharose was eluted with 10 mM NaOH (pH 12.0) and the fractions were analysed for radioactivity. Results are depicted in fig. 4.

## Claims

1. A kit, comprising
a) an antibody or a fragment thereof or a derivative of said antibody or fragment, which antibody or fragment recognizes a target associated antigen, and
b) a ligand-effector moiety conjugate comprising a ligand specific for said antibody, and an effector moiety linked to the ligand,
characterised in that:
- said antibody is a mono specific antibody with multiple binding sites,
- said ligand is derived from the target associated antigen or a cross-reactive analogue thereof recognisable by said antibody.

2. A kit according to claim 1, characterised in that said ligand comprises the cognate epitope.

3. A kit according to claim 1 or 2, characterised in that said ligand comprises a multimere of the cognate epitope or a cross-reactive analogue thereof.

4. A kit according to any of the claims 1 to 3, characterised in that said antibody is a monoclonal antibody, preferably a human monoclonal antibody, more preferably an antibody of the IgM isotype, most preferably the human monoclonal antibody 16.88, deposited with the American Type Culture Collection under number HB 8495.

5. A kit according to claim 4, characterised in that said ligand comprises the cognate epitope of human monoclonal antibody 16.88.

6. A kit according to claim 4 or 5, characterised in that the ligand comprises a multimere of the cognate epitope of the MoAb 16.88, or a peptide analogue of the cognate epitope of the MoAb 16.88.

7. A kit according to claim 4 or 5, characterised in that ligand comprises the epitope of claim 5, having the amino acid sequence -Thr-Leu-Gln-Gly-Leu-Glu-Ile-Glu-Leu-Gln-Ser-Gln-Leu-Ser-Met-Lys-, a fragment thereof or a functional equivalent thereof.

8. A kit according to any of the claims 1-7, characterised in that the effector moiety is a diagnostic agent, preferably a γ-emitting radioisotope, most preferably a radioisotope selected from the group consisting of ^{99m}Tc, ¹¹¹In and ¹²³I.

9. A kit according to any of the claims 1-7, characterised in that the effector moiety is a therapeutic agent, preferably selected from the group consisting of β- and α-emitting radioisotopes, drugs, toxins, boron addends, photosensitizers and radio-sensitizers.

10. A kit according to claim 9, characterised in that the therapeutic radioisotope is selected from the group consisting of ⁹⁰Y, ¹³¹I, ¹⁸⁶Re, ¹⁸⁸Re, ²¹¹At, ²¹²Bi, ²¹³Bi and ²²⁵Ac.

11. A kit according to any of the claims 9-10 characterized in that a diagnostic agent is attached to said antibody, said diagnostic agent preferably being a γ-emitting radioisotope, more preferably selected from the group consisting of ^{99m}Tc, ¹¹¹In and ¹²³I.

12. A ligand-effector moiety conjugate for therapeutical use comprising a ligand derived from a target associated antigen, preferably an epitope, or a cross-reactive analogue thereof, and an effector moiety linked to said ligand.

13. A ligand-effector moiety conjugate according to claim 12, characterised in that said ligand is a multimere of an epitope or a cross reactive analogue thereof.

14. A ligand-effector moiety conjugate according to claim 12 or 13, characterised in that said ligand is the epitope recognised by human monoclonal antibody 16.88.

15. A ligand-effector moiety conjugate according to any of the claims 12-14, characterised in that said effector moiety is a therapeutic agent, preferably selected from the group consisting of β- or α-emitting radioisotopes, drugs, toxins, boron addends, photosensitizers and radiosensitizers.

16. A ligand-effector moiety conjugate according to claim 15, characterised in that the therapeutic radio-isotope is selected from the group consisting of ⁹⁰Y, ¹³¹I, ¹⁸⁶Re, ¹⁸⁸Re, ²¹¹At, ²¹²Bi, ²¹³Bi and ²²⁵Ac.

17. A ligand-effector moiety conjugate according to any of the claims 12-14, characterised in that the effector moiety is a diagnostic agent, preferably a γ-emitting radioisotope, more preferably selected from the group consisting of ^{99m}Tc, ¹¹¹In and ¹²³I.

18. Pharmaceutical preparation comprising a ligand-effector moiety conjugate according to any of the claims 12-16 and a pharmaceutical acceptable carrier.

19. Use of a ligand effector moiety conjugate according to any of the claims 12-16 for the manufacture of a pharmaceutical preparation for treatment of tissue specific cell disorders, preferably anti-cancer treatment or treatment of inflammatory diseases.

20. Method for pretargeting comprising administration of an antibody or a fragment thereof or a derivative of said antibody or fragment, which antibody or fragment recognizes a target associated antigen, followed by administration of a ligand-effector moiety conjugate comprising a ligand specific for said antibody, and an effector moiety linked to the ligand, characterized in that:
- said antibody is a mono specific antibody with multiple binding sites,
- said ligand is derived from the target associated antigen or a cross-reactive analogue thereof recognisable by said antibody.
